# EUROPÄISCHE PATENTSCHRIFT

(11) **EP 0 678 280 B1**
(45) Veröffentlichungstag und Bekanntmachung des Hinweises auf die Patenterteilung: **17.11.1999**
(21) Anmeldenummer: 95103432.1
(22) Anmeldetag: 09.03.1995
(51) Int. Cl.: A61C 1/00

(54) **Zahnärztliche Einrichtung mit ein oder mehreren unterschiedlich konfigurierten Instrumenten**
Dental appliance with one or more variously configured instruments
Appareil dentaire pourvu d'un ou plusieurs instruments de configurations différentes

(30) Priorität: 22.03.1994 DE 4409862
(43) Veröffentlichungstag der Anmeldung: 25.10.1995
(73) Patentinhaber: Sirona Dental Systems GmbH, 64625 Bensheim (DE)
(72) Erfinder: Fornoff, Peter, Dipl. Ing., D-64385 Reichelsheim (DE); Sauer, Uwe, Dipl. Inform., D-67549 Worms (DE); Pabst, Josef, Dipl. Ing., D-68542 Heddesheim (DE); Schulze-Ganzlin, Ulrich, Dipl. Ing., D-64653 Lorsch (DE)
(74) Vertreter: Isenbruck, Günter, Dr.

(56) Entgegenhaltungen:
- WO-A-89/05613
- US-A- 4 180 812

## Beschreibung

Die Erfindung bezieht sich auf eine zahnärztliche Einrichtung nach dem Oberbegriff des Anspruches 1.

Eine derartige Einrichtung ist beispielsweise aus EP-B1-0 391 967 bekannt. Die bekannte Einrichtung enthält eine Anzahl Halter für dynamische, d.h. mit Antriebseinrichtungen versehene Instrumente, deren Bedien- und Betriebsfunktionen sich auf einem Überwachungs- und Steuerpaneel aufzeigen lassen. Hierzu ist ein Mikroprozessor vorhanden, der so konzipiert ist, daß auf dem Paneel eine Anzahl von Funktions-Displayfeldern zum visuellen Aufzeigen einerseits von instrumentenbezogenen Bedienfunktionen und andererseits von Hilfsfunktionen generiert werden kann. An den Mikroprozessor kann außerdem eine alphanumerische Tastatur angeschlossen werden, mit der es möglich ist, eingegebene Informationen auf den Displayfeldern aufzuzeigen. Das Überwachungs- und Steuerpaneel kann ein Bildschirm sein, der zum Aufzeigen von Text- und Videoinformationen eingesetzt werden kann.

Die Erfindung geht davon aus, daß bei einer derartigen zahnärztlichen Einrichtung außer den bereits angesprochenen dynamischen Instrumenten (Bohr- oder Schleifinstrumente), die primär der Präparation dienen, auch bilderfassende Instrumente, z.B. Instrumente mit integrierter Videokamera für intra- oder extraorale Betrachtungen im Patientenmund, zur Anwendung kommen können.

Die Instrumente können außerdem unterschiedlich konfiguriert sein. Dies bedeutet beispielsweise, daß sie mit verschiedenen Aufsätzen (Getriebe-, Objektiv-Aufsätzen oder dergleichen) versehen sein können, oder daß in ihnen unterschiedliche Medien (z.B. mit/ohne Licht, mit/ohne Spray) angeboten bzw. bereit gestellt werden.

Der im Patentanspruch 1 angegebenen Erfindung liegt die Aufgabe zugrunde, eine Einrichtung der eingangs genannten Gattung dahingehend zu verbessern, daß außer den üblichen Präparations-Instrumenten weitere, zur Diagnose dienende bilderfassende Instrumente, die bisher ausschließlich in autarken, peripheren Geräten eingebunden waren, in gleicher Weise wie die vorgenannten Präparationsinstrumente, mit wirtschaftlich vertretbarem Aufwand betrieben werden können.

Mit der vorliegenden Erfindung wird eine Möglichkeit aufgezeigt, wie periphere Geräte, die auf die besonderen Bedingungen und Anforderungen wie Hygiene, Ergonomie, Patientenschutz, etc., zugeschnitten sind und die bisher als autarke Geräte an einem zahnärztlichen Arbeitsplatz angeordnet waren, durch Systemeinbindung an einen Personal-Computer (PC) durch neue oder andere Funktionen profitieren können.

Gemäß einer vorteilhaften Ausgestaltung der Erfindung, bei der die Einrichtung den Instrumenten zugeordnete Ablagehalterungen sowie diesen zugeordnete Positionsmeldesensoren enthält, wie sie beispielsweise aus der US-PS 4 308 011 bekannt sind, können Mittel vorhanden sein, die den Betriebszustand eines instrumentenbezogenen Antriebes, Abweichungen von Soll-Betriebszuständen oder andere Fehlermeldungen dem PC melden und ggf. am Monitor anzeigen. So kann beispielsweise die Entnahme einer IntraoralVideokamera dem PC angezeigt werden, welcher wiederum selbständig das entsprechende Programm aktiviert. Über Bedien- bzw. Schaltelemente an der Kamera oder auch an einem Fußschalter kann der Benutzer sich durch das Programm bewegen und entsprechende Funktionen auslösen. Sobald das Instrument wieder in seine Ablage zurückgelegt wird, wird der ursprüngliche Zustand wieder hergestellt. Wie bereits angesprochen, können periphere Informationen, insbesondere auch Fehlermeldungen, über den Logik-Schaltkreis mitgeteilt und am PC-Monitor angezeigt werden.

Obgleich an sich jede übliche Schnittstelle (ISA-Bus für Einsteckkarten, parallele oder serielle Schnittstelle, Multifunktionsschnittstelle) genutzt werden kann, ist es vorteilhaft, die serielle Schnittstelle für die Datenübertragung heranzuziehen.

Weitere vorteilhafte Ausgestaltungen und Weiterbildungen der Erfindung ergeben sich aus den Patentansprüchen sowie dem nachfolgend anhand der Zeichnung näher erläuterten Ausführungsbeispiel. Es zeigen:
- Fig. 1: eine Prinzipdarstellung einer zahnärztlichen Einrichtung gemäß der Erfindung, und
- Fig. 2: ein Blockschaltbild vom Logik-Schaltkreis.

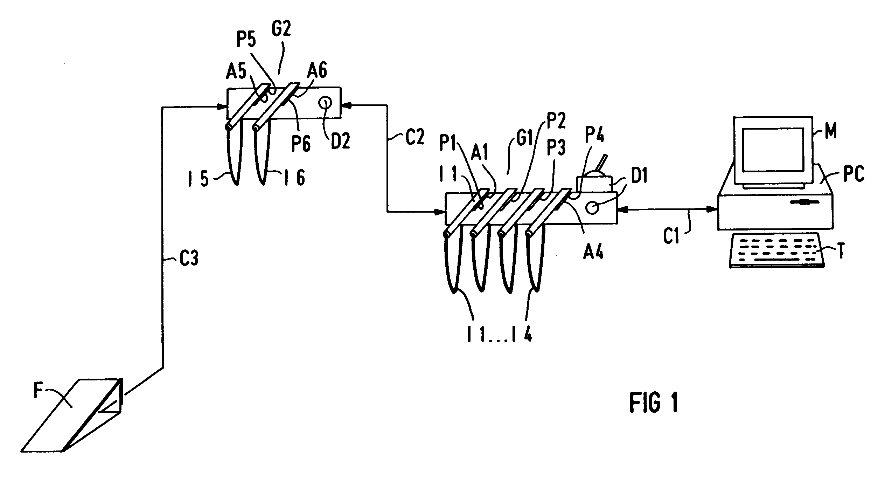

Die Fig. 1 zeigt in einer Prinzipdarstellung eine zahnärztliche Einrichtung mit einem ersten peripheren Gerät G1, welches vier Ablagevorrichtungen A1 bis A4 für dort abgelegte, schlauch- und/oder kabelgebundene Instrumente I1 bis I4 enthält. Mit G2 ist ein zweites peripheres Gerät bezeichnet, welches zwei weitere, in Ablagen A5, A6 gehalterte Instrumente I5 und I6 enthält. Die Instrumente I1 bis I4 können beispielsweise übliche, zur Zahnpräparation geeignete Bohr- und Schleifinstrumente sein, während die Instrumente I5 und I6 bilderfassende Instrumente, beispielsweise Instrumente mit integrierter Videokamera oder Röntgenkamera sein können. Das Gerät G1 ist über ein Kabel C1 mit der seriellen Schnittstelle eines Personalcomputers (PC) verbunden, der in bekannter Weise eine Tastatur T sowie einen Monitor M enthält. Das Gerät G1 enthält einen später noch näher erläuterten Elektronikbaustein mit einem Logik-Schaltkreis, der über ein weiteres Verbindungskabel C2 mit einem weiteren solchen Elektronikbaustein im Gerät G2 verbunden ist. Von diesem führt wiederum ein weiteres Verbindungskabel C3 zu einem mit F bezeichneten Fußschalter.

Sämtlichen Instrumenten I1 bis I6 sind Sensoren in Form von Positionsmeldern P1 bis P6 zugeordnet, welche den Entnahme- bzw. Ablagezustand der Instrumente erfassen. Solche Positionsmelder können Lichtschranken sein, wie sie z.B. in der US-PS 4 308 011 beschrieben sind. Die Positionsmelder geben ein entsprechendes Signal an den vorerwähnten Logik-Schaltkreis, der wiederum ein Informationssignal an den PC weitergibt, welches dann ggf. am Monitor angezeigt werden kann.

Der Fußschalter F besitzt vorzugsweise mehrere aus einer Mittelstellung heraus in zwei entgegengesetze Richtungen verstellbare Schaltpositionen.

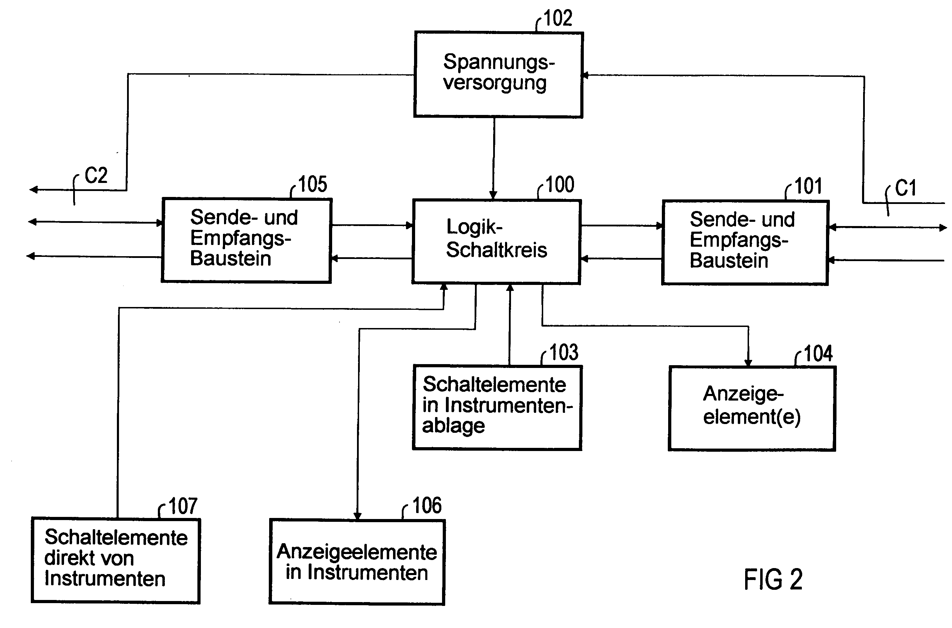

Der schaltungstechnische Aufbau der PC-kommunizierenden Instrumentenablage wird anhand des Blockschaltbildes in Fig. 2 erläutert.

Über einen Logik-Schaltkreis 100, der vorzugsweise ein programmierter IC sein kann, werden alle ein- und ausgehenden Logikzustände geschaltet. Der Logik-Schaltkreis 100 ist über einen Sende- und Empfangsbaustein 101 mittels Kabel C1 mit der Standardschnittstelle des PC's (Fig. 1) verbunden. Die Spannungsversorgung 102 für den Schaltkreis erfolgt vorzugsweise über die serielle Schnittstelle vom PC aus, da diese standardmäßig zur Verfügung steht. Der Logik-Schaltkreis 100 erhält Signale von Schaltelementen 103, die sich in den Instrumentenablagen A1 bis A6 befinden. Im vorliegenden Fall sind dies die Positionsmelder P1 bis P6, die den Ablage- bzw. Entnahmezustand eines Instruments melden. Über den Sende- und Empfangsbaustein 101 werden die Signale zum PC gesendet. Der Logik-Schaltkreis 101 gibt Signale vom PC an Anzeigeelemente 104, die in Fig. 1 mit D1 und D2 bezeichnet sind und am peripheren Gerät G1 oder G2 vorhanden sein können.

Wenn, wie im Ausführungsbeispiel dargestellt, ein zweites peripheres Gerät (G2) vorhanden ist, ist es vorteilhaft, die Instrumentenablagen A5 und A6 hintereinander kaskadiert an die Schnittstelle des PC's anzuschließen. Im aufgezeigten Anwendungsfall enthält dann auch das Gerät G2 einen entsprechenden, in Fig. 2 nicht dargestellten Logik-Schaltkreis, wie in Fig. 2 dargestellt, der über das Kabel C2 mit dem Logik-Schaltkreis 100 im Gerät G1 verbunden ist. Ebenso ist ein weiterer Sende- und Empfangsbaustein 105 vorgesehen, der die gleiche Funktion erfüllt, wie der Baustein 101. Mit 106 sind Anzeigeelemente bezeichnet, die ebenfalls über den Logikschaltkreis aktiviert werden und die Betriebsbereitschaft der Instrumente, Fehlermeldungen oder auch Statusfunktionen, wie Drehzahl, anzeigen. Mit 107 sind Schaltelemente bezeichnet, die die Instrumente identifizieren. Diese Schaltelemente können im Instrument selbst oder auch im daran angeschlossenen Versorgungsschlauch angeordnet sein. Die von diesen Schaltelementen ausgehenden Signale werden zunächst an den Logik-Schaltkreis 100 und dann über den Sende-Baustein 101 an denn PC weitergeleitet. Diese Anordnung hat den Vorteil, daß nur eine serielle Schnittstelle am PC belegt zu werden braucht.

Nachfolgend werden einige besonders vorteilhafte Anwendungen geschildert:
Bei Entnahme eines Instrumentes I1 bis I4 aus seiner Halterung A1 bis A4 wird über den Logik-Schaltkreis das Anwendungsprogramm im PC, das jeweils dem gezogenen Instrument zugeordnet ist, aktiviert. Wird beispielsweise ein Bohrinstrument mit einem im Instrument angeordneten Elektromotorantrieb aus seiner Ablage entnommen, so wird das diesem Instrument zugeordnete Steuer- und/oder Überwachungsprogramm im PC aktiviert. Bei Ablage des Instrumentes wird ein neutraler Zustand eingestellt. Wird danach ein anderes Instrument, beispielsweise mit integrierter Videokamera (I5 oder I6), gezogen, so wird, aktiviert durch den im Gerät G2 vorhandenen Logik-Schaltkreis, im PC das Programm auf das dem jetzt gezogenen Instrument zugeordnete Instrumentenprogramm umgeschaltet.

Unter Bezugnahme auf das abgespeicherte Programm kann in dem erwähnten Fall beispielsweise ein am Instrument oder auch am Fußschalter F vorhandener Auslöseschalter ein bestimmtes Videobild "einfrieren".

Mit dem Fußschalter F, der mehrere Schaltpositionen aufweist, können beispielsweise im Anwenderprogramm des PC's einzelne Instrumentenprogramme ausgewählt und aktiviert werden.

Im Rahmen der Erfindung ist es möglich, die vorhandene Funktion des Auslöseschalters am Fußschalter zu erweitern, indem man der zeitlichen Abfolge des Auslösens eine zusätzliche Bedeutung zukommen läßt. So kann beispielsweise durch einfaches Klicken, Doppelklicken oder Halten des Auslöseschalters die Funktion des Fußschalters erweitert werden. Falls der PC und die die serielle Schnittstelle überwachende Software nicht zur Differenzierung von solchen unterschiedlichen Schaltvorgängen ausgelegt ist, wird dies die Logik-Schaltung im PC übernehmen. Je nach Schaltfolge und Schaltdauer sendet die Logik-Schaltung über die serielle Schnittstelle ein codiertes Datenwort an den PC, welcher über einen an sich bekannten Codeschlüssel von der Schaltaktion informiert wird. Üblicherweise ist ein solches Datenwort sieben bis acht Bit lang. Dieses Datenwort kann bei jeder neuen Aktion zum PC gesendet werden.

## Patentansprüche

1. Zahnärztliche Einrichtung, enthaltend ein oder mehrere, in Ablagevorrichtungen (A1 bis A4) gehalterte Präparationsinstrumente (I1 bis I4) unterschiedlicher Konfiguration, denen Sensoren (P1 bis P4) zugeordnet sind, die den Konfigurations- und/oder Ablagezustand der Präparationsinstrumente erfassen, und eine den Präparationsinstrumenten zugeordnete Steuereinrichtung, die einen Mikroprozessor, eine Tastatur und einen Bildschirm umfaßt, mit deren Hilfe sich instrumentenbezogene Funktionen in Abhängigkeit von einer Entnahme bzw. Ablage eines Instruments am Bildschirm anzeigen und gegebenenfalls mit Hilfe eines Fußschalters (F) aktivieren lassen, **dadurch gekennzeichnet**, daß den Präparationsinstrumenten (I1 bis I4) und weiteren, zur Diagnose dienenden bilderfassenden Instrumenten (I5, I6), ein Personal-Computer (PC) mit Tastatur (T) und Bildschirm (M) gemeinsam ist, der den Mikroprozessor enthält und der mehrere Schnittstellen aufweist, über deren eine Schnittstelle Daten aus instrumentenbezogenen Funktionen ein- und auslesbar sind, wobei im Personal-Computer (PC) mehrere, den unterschiedlichen Instrumenten zugeordnete Anwendungsprogramme installiert sind, und daß ein Logik-Schaltkreis (100) vorhanden ist, der einerseits mit der einen Schnittstelle des PCs und andererseits mit Schalt-und Anzeigeelementen (103, 104, 106, 107) verbunden ist, wobei über den Logik-Schaltkreis (100) und der Schnittstelle des PCs bidirektionale Betriebsdaten übertragen werden, die den Instrumenten bzw. den Instrumenten zugeordneten Sensoren entsprechen, und wobei der Logik-Schaltkreis (100) in Abhängigkeit von einem aus einer Ablagevorrichtung (A1 bis A4) entnommenen Instrument den Personal-Computer (PC) dazu aktiviert, das dem entnommenen Instrument zugeordnete Anwendungsprogramm bereitzustellen.

2. Zahnärztliche Einrichtung nach Anspruch 1, **dadurch gekennzeichnet,** daß Mittel vorhanden sind, die den Betriebszustand eines instrumentenbezogenen Antriebes auf dem Bildschirm (M) anzeigen.

3. Zahnärztliche Einrichtung nach Anspruch 2, **dadurch gekennzeichnet,** daß das Anwendungsprogramm eines Instruments so ausgeführt ist, daß auf dem Bildschirm (M) Abweichungen von Soll-Betriebswerten des Antriebes angezeigt werden.

4. Zahnärztliche Einrichtung nach einem der Ansprüche 1 bis 3, **dadurch gekennzeichnet,** daß ein Fußschalter (F) vorgesehen ist, der mehrere Schaltpositionen aufweist mit denen sich über den Logik-Schaltkreis (100) die Anwendungsprogramme vorzugsweise die instrumentenbezogenen Anwendungsprogramme, anwählen lassen.

5. Zahnärztliche Einrichtung nach einem der Ansprüche 1 bis 4, **dadurch gekennzeichnet,** daß der Logik-Schaltkreis (100) an eine serielle Schnittstelle des PC's angeschlossen ist.

6. Zahnärztliche Einrichtung nach einem der Ansprüche 1 bis 5, **dadurch gekennzeichnet,** daß ein oder mehrere Instrumente (I5, I6) hintereinander kaskadiert an der Schnittstelle des PC's angeschlossen sind.

7. Zahnärztliche Einrichtung nach Anspruch 6, **dadurch gekennzeichnet,** daß mehrere Instrumente zu Gruppen (I1 bis I4; I5, I6) zusammengefaßt sind, wobei jeder Gruppe ein Logik-Schaltkreis zugeordnet ist.

8. Zahnärztliche Einrichtung nach einem der Ansprüche 1 bis 7, **dadurch gekennzeichnet,** daß der Logik-Schaltkreis so ausgebildet ist, daß er die Art der zeitlichen Aktivierung der Schalttelemente erkennt und in einen PC-erkennbaren Code umsetzt und überträgt, wobei die Informationen in codierter Form bidirektional übertragen werden.

## Claims

1. Dental appliance, containing one or more variously configured preparation instruments (I1 to I4) which are retained in depositing devices (A1 to A4) and are assigned sensors (P1 to P4), which detect the configuration and/depositing state of the preparation instruments, and a control apparatus assigned to the preparation instruments and comprising a microprocessor, a keyboard and a screen, with the aid of which instrument-related functions can be displayed on the screen, and if appropriate activated with the aid of a foot switch (F), in dependence on removal or depositing of an instrument, characterized in that the preparation instruments (I1 to I4) and further, image-acquiring instruments (I5, I6), which are used for diagnosis, share a personal computer (PC) with keyboard (T) and screen (M) which contains the microprocessor and has a plurality of interfaces, via one interface of which data from instrument-related functions can be read in and out, a plurality of application programs assigned to the various instruments being installed in the personal computer (PC), and in that a logic circuit (100) is present, which is connected to the one interface of the PC, on the one hand, and to switching and display elements (103, 104, 106, 107), on the other hand, bidirectional operating data corresponding to the instruments or sensors assigned to the instruments being transferred via the logic circuit (100) and the interface of the PC, and the logic circuit (100), in dependence on an instrument removed from a depositing device (A1 to A4), activating the personal computer (PC) in respect of providing the application program assigned to the instrument removed.

2. Dental appliance according to Claim 1, characterized in that means are present which display the operating state of an instrument-related drive on the screen (M).

3. Dental appliance according to Claim 2, characterized in that the application program of an instrument is designed in such a way that deviations from desired operating values of the drive are displayed on the screen (M).

4. Dental appliance according to one of Claims 1 to 3, characterized in that a foot switch (F) is provided, which has a plurality of switching positions which enable the application programs, preferably instrument-related application programs, to be selected via the logic circuit (100).

5. Dental appliance according to one of Claims 1 to 4, characterized in that the logic circuit (100) is connected to a serial interface of the PC.

6. Dental appliance according to one of Claims 1 to 5, characterized in that one or more instruments (I5, I6) are connected to the interface of the PC such that they are cascaded in series.

7. Dental appliance according to Claim 6, characterized in that a plurality of instruments are combined into groups (I1 to I4; I5, I6), each group being assigned a logic circuit.

8. Dental appliance according to one of Claims 1 to 7, characterized in that the logic circuit is designed in such a way that it identifies the type of temporal activation of the switching elements and converts it into a PC-identifiable code and transfers it, the information being transferred bidirectionally in coded form.

## Revendications

1. Appareil de dentiste, contenant un ou plusieurs instruments de préparation (I1 à I4) de configurations différentes, qui sont placés dans des dispositifs de rangement (A1 à A4) et auxquels sont associés des capteurs (P1 à P4) qui détectent l'état de configuration et/ou de rangement des instruments de préparation, et un dispositif de commande, qui est associé aux instruments de préparation, qui comprend un microprocesseur, un clavier et un écran et à l'aide duquel des fonctions relatives aux instruments peuvent être affichées sur l'écran en fonction de l'enlèvement ou du rangement d'un instrument et peuvent éventuellement être activées à l'aide d'un interrupteur à commande au pied (F), caractérisé en ce qu'il est associé en commun aux instruments de préparation (I1 à I4) et à d'autres instruments (I5, I6) servant à acquérir des images pour établir un diagnostic, un ordinateur personnel (PC) avec clavier (T) et écran (M) qui contient le microprocesseur et qui comporte plusieurs interfaces, des données concernant des fonctions relatives aux instruments pouvant être lues et écrites par l'intermédiaire de l'une de ces interfaces et plusieurs programmes d'application associés aux différents instruments étant installés dans l'ordinateur personnel (PC), et en ce qu'il est prévu un circuit logique (100) qui est relié d'une part à ladite interface du PC et d'autre part à des éléments de commutation et d'affichage (103, 104, 106, 107), des données d'exploitation bidirectionnelles qui correspondent aux instruments ou à des capteurs associés aux instruments étant transmises par l'intermédiaire du circuit logique (100) et de l'interface du PC et le circuit logique (100) activant l'ordinateur personnel (PC) en fonction d'un instrument enlevé d'un dispositif de rangement (A1 à A4) pour que cet ordinateur personnel mette à disposition le programme d'application associé à l'instrument enlevé.

2. Appareil de dentiste selon la revendication 1, caractérisé en ce qu'il est prévu des moyens qui affichent sur l'écran (M) l'état d'exploitation d'un dispositif d'entraînement relatif aux instruments.

3. Appareil de dentiste selon la revendication 2, caractérisé en ce que le programme d'application d'un instrument est conçu de telle sorte que des écarts par rapport à des valeurs d'exploitation de consigne du dispositif d'entraînement sont affichés sur l'écran (M).

4. Appareil de dentiste selon l'une des revendications 1 à 3, caractérisé en ce qu'il est prévu un interrupteur à commande au pied (F) qui comporte plusieurs positions de commutation avec lesquelles on peut sélectionner par l'intermédiaire du circuit logique (100) les programmes d'application, de préférence les programmes d'application relatifs aux instruments.

5. Appareil de dentiste selon l'une des revendications 1 à 4, caractérisé en ce que le circuit logique (100) est raccordé à une interface série du PC.

6. Appareil de dentiste selon l'une des revendications 1 à 5, caractérisé en ce qu'un ou plusieurs instruments (I5, I6) sont raccordés en cascade à l'interface du PC.

7. Appareil de dentiste selon la revendication 6, caractérisé en ce que plusieurs instruments sont rassemblés en groupes (I1 à I4 ; I5, I6), un circuit logique étant associé à chaque groupe.

8. Appareil de dentiste selon l'une des revendications 1 à 7, caractérisé en ce que le circuit logique est conçu de telle sorte qu'il reconnaît le type d'activation chronologique des éléments de commutation, qu'il le convertit en un code reconnaissable par un PC et qu'il transmet celui-ci, les informations étant transmises de façon bidirectionnelle sous forme codée.
